# EUROPÄISCHE PATENTANMELDUNG

(11) **EP 0 945 424 A2**
(43) Veröffentlichungstag der Anmeldung: **29.09.1999**
(21) Anmeldenummer: 99104525.3
(22) Anmeldetag: 06.03.1999
(51) Int. Cl.: C07C 69/88, C07C 67/08

(54) **Verfahren zur Herstellung von Hydroxybenzoesäureestern von Oxoalkoholen**

(30) Priorität: 26.03.1998 DE 19813338
(71) Anmelder: HÜLS AKTIENGESELLSCHAFT, 45764 Marl (DE)
(72) Erfinder: Köhler, Günther Dr., 45770 Marl (DE); Korell, Michael Dr., 44801 Bochum (DE)
(74) Vertreter: Olbricht, Gerhard, Dr.

(57) **Zusammenfassung**

Die Erfindung betrifft ein Verfahren zur Herstellung von Hydroxybenzoesäureestern von Oxoalkoholen, bei dem man eine Hydroxybenzoesäure mit einem Oxoalkohol in Gegenwart von anderen Stoffen, die in den Reaktionsgemischen der Oxierung von Olefinen enthalten sind, sowie in Gegenwart eines sauren Katalysators verestert oder einen Hydroxybenzoesäureester eines niederen Alkohols unter denselben Bedingungen mit einem Oxoalkohol umestert.

## Beschreibung

Die Erfindung betrifft ein Verfahren zur Herstellung von Hydroxybenzoesäureestern von Oxoalkoholen durch Veresterung von Hydroxybenzoesäuren oder Umesterung von Hydroxybenzoesäureestern niederer Alkohole mit Reaktionsgemischen aus der Oxosynthese in Gegenwart eines bestimmten sauren Katalysators.

Ester der Hydroxybenzoesäuren und insbesondere der 4-Hydroxybenzoesäure mit höheren Alkoholen, wie sie durch Oxierung (oder Hydroformylierung) von Olefinen erhältlich sind ("Oxoalkohole"), werden in zunehmendem Maße als Weichmacher für Polymere, insbesondere für Polyamide und Polyester, verwendet.

Bekannt sind Verfahren zur Herstellung von 4-Hydroxybenzoesäureestern durch Veresterung der freien Säure mit Alkoholen unter Einsatz von Lösemitteln, wie Benzol (V.Carill, J.Chem.Soc.ind.London 66 (1947) 175, 176), Aceton (JP 53112-634) oder Dioxan (JP 77048-966). Dabei wurden reine, zuvor destillierte Alkohole eingesetzt. Die Lösemittel dienten in diesen Fällen als Verdünnungsmittel zur Sicherstellung der Rührbarkeit der Reaktionsgemische und teilweise auch als Schleppmittel für das entstehende Reaktionswasser.

Eine Aufgabe der Erfindung besteht darin, ein Verfahren zur Herstellung von Estern der Hydroxybenzoesäuren mit Oxoalkohole bereitzustellen, bei dem keine reinen, zuvor destillierten Alkohole eingesetzt werden müssen. Eine andere Aufgabe der Erfindung besteht darin, ein Verfahren zur Herstellung solcher Ester bereitzustellen, das keinen Zusatz von Lösemitteln erfordert.

Erfindungsgemäß wurden diese Aufgaben gelöst durch ein Verfahren zur Herstellung von Hydroxybenzoesäureestern von Oxoalkoholen, bei dem man eine Hydroxybenzoesäure mit einem Oxoalkohol in Gegenwart von anderen Stoffen, die in den Reaktionsgemischen der Oxierung von Olefinen enthalten sind, sowie in Gegenwart eines sauren Katalysators verestert oder einen Hydroxybenzoesäureester eines niederen Alkohols unter denselben Bedingungen mit einem Oxoalkohol umestert.

Bei dem erfindungsgemäßen Verfahren wirken die in den Reaktionsgemischen der Oxierung enthaltenen anderen Stoffe zugleich als Verdünnungsmittel für das Reaktionsgemisch und als Schleppmittel für das Reaktionswasser. Durch den Einsatz von Reaktionsgemischen der Oxo-Synthese anstelle von reinen Oxoalkoholen und den Verzicht auf den Zusatz von Lösemitteln, wie sie nach dem Stand der Technik verwendet werden, lassen sich die Verfahrenskosten deutlich reduzieren.

Die bevorzugte Hydroxybenzoesäure ist die 4-Hydroxybenzoesäure, die durch Oxidation von p-Kresol im technischen Maßstab zugänglich ist. Hydroxybenzoesäureester niederer Alkohole, insbesondere mit C₁-C₄-Alkanolen, sind durch Veresterung der Säuren mit einem Überschuß an dem betreffenden niederen Alkanol zugänglich. Die Alkanole müssen auf jeden Fall niedriger sieden als die Oxoalkohole, deren Ester hergestellt werden sollen. Die direkte Veresterung der Hydroxybenzoesäuren mit Oxoalkoholen wird gegenüber der Umesterung bevorzugt.

Bevorzugte Dxoalkohole enthalten 7 bis 21 Kohlenstoffatome. Sie werden aus Olefinen mit 6 bis 20 Kohlenstoffatomen durch Umsetzung mit Kohlenmonoxid und Wasserstoff bei erhöhter Temperatur und unter erhöhtem Druck in Gegenwart von Kobalt- oder Rhodiumkatalysatoren ("Oxierung") hergestellt. Die Ausgangsolefine sind vielfach isomere Oligomere von niederen Olefinen, wie Dibuten, Tripropylen, Tetrapropylen und Tetrabuten. Die Oxoalkohole sind dementsprechend ebenfalls Isomerengemische. Es ist ein wesentliches Merkmal des Verfahrens nach der Erfindung, daß die Oxoalkohole in Gegenwart von Stoffen, die in den Reaktionsgemischen der Oxierung von Olefinen enthalten sind, für die Veresterung bzw. Umesterung eingesetzt werden. Dabei handelt es sich vor allem um Kohlenwasserstoffe, die als Nebenprodukte der Oxierungsreaktion durch Hydrierung der Ausgangsolefine und/oder durch Dehydratisierung der Oxoalkohole entstehen. Zweckmäßig setzt man die rohen Reaktionsgemische aus der Oxoreaktion unmittelbar für das Verfahren nach der Erfindung ein. Es ist aber auch möglich, die Reaktionsgemische zuvor anzudestillieren, d.h. einen Teil der niedriger als die Oxoalkohole siedenen Bestandteile zu entfernen. Weiterhin kann man vorteilhaft "abgetoppte" Oxoalkohole einsetzen, d.h. durch einfache Destillation vom Katalysator und von Hochsiedern abgetrennte Oxoreaktionsgemische.

Als Veresterungs- bzw. Umesterungskatalysator verwendet man besonders vorteilhaft ein natürliches Aluminiumhydrosilikat, das gegebenenfalls durch Behandlung mit einer Mineralsäure, vorteilhaft mit konzentrierter oder verdünnter (z.B. 5- bis 20-%iger) Salzsäure, in seine Säureform überführt wurde. Es hat sich gezeigt, daß diese Katalysatoren die besten Ausbeuten und Raum-Zeit-Ausbeuten ergeben. Ein anderer wichtiger Vorteil dieser Katalysatoren liegt darin, daß man auch ohne Destillation praktisch farblose Ester erhält, wie sie für die Verwendung als Weichmacher benötigt werden. Übliche Ver- bzw. Umesterungskatalysatoren, wie Schwefelsäure, p-Toluolsulfosäure oder Titanate, ergeben dagegen deutlich gefärbte Produkte, die nicht unmittelbar als Weichmacher geeignet sind. Weiterhin ist natürlich vorteilhaft, daß die erfindungsgemäßen Katalysatoren als Feststoffe leicht aus dem Reaktionsgemisch abgetrennt werden können. Bevorzugte Katalysatoren sind die natürlichen Aluminiumhydrosilikate vom Montmorrillonit-Typ, die mit konzentrierter oder verdünnter Salzsäure sauer eingestellt wurden. Ein geeignetes Aluminiumhydrosilikat ist z.B. der Montmorrillonit KS der Südchemie AG, D-85368 Moosburg.

Man wendet die Hydroxybenzoesäure und den Oxoalkohol im allgemeinen in etwa stöchiometrischen Mengen oder mit einem Überschuß an Oxoalkohol an, beispielsweise von bis zu 20 Molprozent. Der Katalysator wird zweckmäßig in Mengen von 1 bis 5 Gew.-%, eingesetzt, bezogen auf das gesamte Reaktionsgemisch. Die Reaktionstemperatur beträgt zweckmäßig 120 bis 200°C. Man kann bei Atmosphärendruck arbeiten. Verminderter Druck fördert die Entfernung von Wasser aus dem Reaktionsgemisch und verkürzt die Reaktionszeit, die im allgemeinen 2 bis 5 Stunden beträgt.

Die Veresterung bzw. Umesterung kann diskontinuierlich durchgeführt werden, beispielsweise indem man das Gemisch aus Ausgangsstoffen und Katalysator in einem Rührreaktor erhitzt, die leicht flüchtigen Anteile abdestilliert und zu einem zweiphasigen Gemisch kondensiert, die wässerige oder wäßrig-alkoholische Phase ausschleust und gegebenenfalls die organische Phase in den Rührreaktor zurückführt, bis kein Wasser mehr ausgetragen wird. Aus dem Reaktionsgemisch trennt man den festen Katalysator durch Filtrieren, Absaugen usw. ab, wäscht gegebenfalls den abgetrennten Katalysator, z.B. mit der organischen Phase aus dem Wasserabscheider, und trennt das Filtrat und gegebenenfalls die Waschflüssigkeit durch gegebenenfalls mehrstufige Destillation, vorzugsweise unter Mitverwendung eines Strippgases, wie Stickstoff, in Kohlenwasserstoffe, nicht umgesetzte Oxoalkohole, gegebenenfalls auch Hydroxybenzoesäure und die gewünschten Hydroxybenzoesäureester der Oxoalkohole. Vorteilhaft führt man die Destillation in einem Dünnschichtverdampfer durch. Die Hydroxybenzoesäureester der Oxoalkohole fallen als Destillationsrückstand mit einer gaschromatographisch bestimmten Reinheit von >98% in einer Ausbeute an, die 90% und mehr, bezogen auf eingesetzte Hydroxybenzoesäure, betragen kann. Wenn man von einem Oxoalkoholgemisch ausgegangen ist, das noch den Oxo-Katalysator enthält, kann man den erhaltenen Ester abdestillieren und aus dem Rückstand, z.B. durch Säureextraktion, den Katalysator bzw. dessen Wertstoffe zurückgewinnen, wie dies in der Oxoreaktion üblich ist.

Die folgenden Beispiele sollen die Erfindung weiter erläutern, nicht aber deren Anwendungsbereich begrenzen, wie er in den Patentansprüchen dargelegt ist.

### Beispiele

### Beispiel 1

1.308g rohes i-Heptadecanol (ca. 70%, entsprechend 3,6 mol i-Heptadecanol; direkt aus der Oxosynthese stammend), 414g (3,0 mol) 4-Hydroxybenzoesäure und 42 g eines sauren Aluminiumhydrosilikats vom Montmorrillonit-Typ (Montmorillonit KS der Südchemie AG) werden in einem 2.5 l-Kolben mit Rührer, Rückflußkühler (0 bis 5°C) und Wasserabscheider in einer Stickstoffatmosphäre gemischt und etwa 30 min bei Raumtemperatur gerührt, um die flüssige Phase von Sauerstoff zu befreien. Anschließend wird das Gemisch unter Einleiten eines Stickstoffstromes auf 170°C erhitzt. Innerhalb von 3 h scheiden sich 132g eines zweiphasigen Gemisches ab. Nach Abkühlen auf 40°C wird das Reaktionsgemisch über eine Druckfilterpresse filtriert. Es werden 112,3g Feststoff sowie 1.365,5g Filtrat erhalten. Das Filtrat wird zweistufig am Dünnschichtverdamper destilliert. Zunächst werden bei 170°C/100mbar die Leichtsieder entfernt, wobei ein Sumpfprodukt von 1.247,1g erhalen wird. In einem zweiten Durchgang werden aus dem Sumpfprodukt bei 170°C/80mbar (volles Vakuum der Drehschieberpumpe, 80mbar durch Stickstoff eingestellt) die restlichen unter diesen Bedingungen destillierbaren Bestandteile abdestilliert. Es verbleiben als zweites Sumpfprodukt 927,0g Produkt, das nach GC-Analyse 98,9% 4-Hydroxybenzoesäureisoheptadecylester enthält. Die Ausbeute beträgt 81,3%, bezogen auf eingesetzte 4-Hydroxybenzoesäure.

### Beispiel 2

160,7kg rohes i-Heptadecanol (ca. 70%, entsprechend 437 kmol i-Heptadecanol; direkt aus der Oxosynthese stammend), 50kg (362 kmol) 4-Hydroxybenzoesäure und 3 kg des im Beipiel 1 verwendeten Katalysators werden in einem Rührkessel vorgelegt. Unter Einleiten von Stickstoff (bis zu 5 m³/h) wird die Reaktionsmischung auf 175°C erhitzt, wobei das entstehend Reaktionswasser abdestilliert. Wenn die Säurezahl eine Wert von <5mg KOH/g Probe erreicht, wird der Stickstoffstrom auf 500l/h erhöht und solange bei 175°C weitergerührt, bis die Säurezahl auf <1mg KOH/g Probe gesunken ist. Anschließend wird langsam auf einen Druck von 5hPa evakuiert, um Leichtsieder einschließlich nicht umgesetzten Edukts zu entfernen. Nach dem Abkühlen auf 80°C wird das Reaktionsgemisch mit Stickstoff auf einer Druckfilterpresse vom Katalysator befreit. Es werden 113kg Produkt erhalten, das nach GC-Analyse 99,2% 4-Hydroxybenzoesäureisoheptadecylester enthält. Die Ausbeute beträgt 82,9%, bezogen auf eingesetzte 4-Hydroxybenzoesäure.

## Patentansprüche

1. Verfahren zur Herstellung von Hydroxybenzoesäureestern von Oxoalkoholen, dadurch gekennzeichnet, daß man eine Hydroxybenzoesäure mit einem Oxoalkohol in Gegenwart von anderen Stoffen, die in den Reaktionsgemischen der Oxierung von Olefinen enthalten sind, sowie in Gegenwart eines sauren Katalysators verestert oder einen Hydroxybenzoesäureester eines niederen Alkohols unter denselben Bedingungen mit einem Oxoalkohol umestert.

2. Verfahren nach Anspruch 1, dadurch gekennzeichnet, daß die Hydroxybenzoesäure 4-Hydroxybenzoesäure ist.

3. Verfahren nach Anspruch 1 oder 2, dadurch gekennzeichnet, daß die Oxoalkohole 7 bis 21 Kohlenstoffatome enthalten.

4. Verfahren nach Anspruch 3, dadurch gekennzeichnet, daß man als Oxoalkohole die rohen Reaktionsgemische aus der Oxoreaktion unmittelbar einsetzt.

5. Verfahren nach Anspruch 3, dadurch gekennzeichnet, daß man als Oxoalkohole das durch Abtoppen vom Oxo-Katalysator und von Hochsiedern gewonnene Oxoreaktionsgemisch einsetzt.

6. Verfahren nach einem der Ansprüche 1 bis 5, dadurch gekennzeichnet, daß man als Veresterungs- bzw. Umesterungskatalysator ein natürliches Aluminiumhydrosilikat verwendet.

7. Verfahren nach Anspruch 6, dadurch gekennzeichnet, daß man ein Aluminiumhydrosilikat vom Montmorrillonit-Typ verwendet.

8. Verfahren nach Anspruch 6 oder 7, dadurch gekennzeichnet, daß das Aluminumhydrosilikat durch Behandlung mit einer Mineralsäure in seine Säureform überführt wurde.

9. Verfahren nach einem der Ansprüche 1 bis 8, dadurch gekennzeichnet, daß die Aufarbeitung des Reaktionsgemisches durch Abtrennen des Katalysators und gegebenenfalls mehrstufige Destillation der flüssigen Phase erfolgt.

10. Verfahren nach Anspruch 9, dadurch gekennzeichnet, daß man bei der Aufarbeitung ein Strippgas und einen Dünnschichtverdampfer mitverwendet.
